# EUROPEAN PATENT SPECIFICATION

(11) **EP 3 854 774 B1**
(45) Date of publication and mention of the grant of the patent: **20.03.2024**
(21) Application number: 20782838.5
(22) Date of filing: 30.03.2020
(51) Int. Cl.: C07C 67/08, C07C 67/48, C07C 67/03, B01J 31/02, B01J 8/00, B01J 19/00

(54) **METHOD FOR PREPARING ESTER-BASED COMPOSITION**
VERFAHREN ZUR HERSTELLUNG EINER ESTERBASIERTEN ZUSAMMENSETZUNG
PROCÉDÉ DE PRÉPARATION D'UNE COMPOSITION À BASE D'ESTER

(30) Priority: 04.04.2019 KR 20190039716
(43) Date of publication of application: 28.07.2021
(73) Proprietor: LG Chem, Ltd., Seoul 07336, (KR)
(72) Inventor: KIM, Hyun Kyu, Daejeon 34122 (KR); LEE, Sung Kyu, Daejeon 34122 (KR); MOON, Jeong Ju, Daejeon 34122 (KR); JUN, Hyoung, Daejeon 34122 (KR); JIN, Chan Hyu, Daejeon 34122 (KR)
(74) Representative: Goddar, Heinz J.
(86) International application number: PCT/KR2020/004375
(87) International publication number: WO 2020/204555

(56) References cited:
- WO-A1-2019/050281
- JP-A- 2009 114 273
- JP-A- 2009 191 205
- KR-A- 20130 042 742
- KR-A- 20130 042 742
- KR-A- 20190 027 623
- KR-B1- 101 663 586
- KR-B1- 101 663 586
- US-A1- 2007 077 635

## Description

### TECHNICAL FIELD

The present invention relates to a method for manufacturing an ester-based composition which is characterized in sequentially operating a plurality of batch reactors.

### BACKGROUND ART

Phthalate-based plasticizers had occupied 92% of the world's plasticizer market by the 20th century (Mustafizur Rahman and Christopher S.Brazel "The plasticizer market: an assessment of traditional plasticizers and research trends to meet new challenges" Progress in Polymer Science 2004, 29, 1223-1248), and are additives used to improve the processability of polyvinyl chloride (hereinafter, referred to as PVC) by imparting flexibility, durability, cold resistance, and the like and lowering viscosity during melting. Phthalate-based plasticizers are introduced into PVC in various contents and used not only for hard products such as rigid pipes, but also for soft products such as food packaging materials, blood bags, and flooring materials since the phthalate-based plasticizers are soft and stretchable. Thus, the phthalate-based plasticizers are more closely related to real life than any other materials and are widely used for materials which come into direct contact with a human body.

However, despite the compatibility with PVC and excellent softness imparting properties of phthalate-based plasticizers, there has been controversy over the harmful nature of the phthalate-based plasticizers in that when a PVC product containing a phthalate-based plasticizer is used in real life, the phthalate-based plasticizer may be leaked little by little out of the product and act as a suspected endocrine disruptor (environmental hormone) and a carcinogen to the level of a heavy metal (NR Janjua et al. "Systemic Uptake of Diethyl Phthalate, Dibutyl Phthalate, and Butyl Paraben Following Whole-body Topical Application and Reproductive and Thyroid Hormone Levels in Humans" Environmental Science and Technology 2008, 42, 7522-7527). Particularly, since a report was published in the 1960s in the United States that diethylhexyl phthalate (di-(2-ethylhexyl) phthalate, DEHP), the most used phthalate plasticizer, leaked out of PVC products, global environmental regulations have started to be implemented in addition to various studies on the harmful nature of the phthalate-based plasticizer on human bodies, boosted by increasing interest in environmental hormones in the 1990s.

Thus, in order to respond to environmental hormonal problems and environmental regulations due to the leakage of phthalate-based plasticizers, many researchers have been conducting research in order to develop a new non-phthalate-based alternative plasticizer without phthalic anhydride used in the manufacturing of phthalate-based plasticizers, or to develop a leakage suppression technology which suppresses the leakage of phthalate-based plasticizers, thereby significantly reducing risks to human bodies and which meets environmental standards.

Meanwhile, as non-phthalate-based plasticizers, terephthalate-based plasticizers not only have an equivalent level of physical properties with phthalate-based plasticizers, but also have been spotlighted as a material free from environmental problems, so that various types of terephthalate-based plasticizers have been developed. In addition, research on developing terephthalate-based plasticizers with excellent physical properties as well as research on equipment for manufacturing such terephthalate-based plasticizers have been actively conducted, and there has been a demand for more efficient, more economical and simpler process designs in terms of process design.

### Prior Art Document

(Patent Document 1) Korean Patent Laid-Open Publication No. 10-1354141

(Non-patent Document 1) Mustafizur Rahman and Christopher S. Brazel "The plasticizer market: an assessment of traditional plasticizers and research trends to meet new challenges" Progress in Polymer Science 2004, 29, 1223-1248

(Non-patent Document 2) N. R. Janjua et al. "Systemic Uptake of Diethyl Phthalate, Dibutyl Phthalate, and Butyl Paraben Following Whole-body Topical Application and Reproductive and Thyroid Hormone Levels in Humans" Environmental Science and Technology 2008, 42, 7522-7527

KR 2013-0042742 A discloses a system and a process for manufacturing an ester-based composition, the system comprising: a mixer in which a reaction mixture of a polycarboxylic acid and an alcohol having 3 to 10 alkyl carbon atoms is formed; a reaction unit provided with N number of batch reactors connected in parallel in which an esterification reactor of the reaction mixture is performed, an inlet line for receiving the reaction mixture from the mixer, and an outlet line for discharging reaction products from the N number of batch reactors; and a separation unit for receiving the reaction products through the outlet line of the reaction unit and removing unreacted alcohol therefrom.

KR 101663586 B1 discloses a method for manufacturing an ester-based composition with control of the flow rate to parallel reactors.

WO 2019/050281 A1 discloses an additional trans-esterification step in a method for manufacturing an ester-based composition.

### DISCLOSURE OF THE INVENTION

### TECHNICAL PROBLEM

An aspect of the present invention, which is defined in claim 1, provides an efficient method for manufacturing an ester-based composition, the method securing the stability of batch reactors and the efficiency of a semi-continuous process by employing a plurality of batch reactors mainly used in an esterification reaction, connecting the plurality of batch reactors in parallel, and sequentially operating the same such that the entire process is operated semi-continuously.

### TECHNICAL SOLUTION

There is provided a method for manufacturing an ester-based composition, the method of claim 1 including a step S1 of injecting a polycarboxylic acid and alcohol having 3 to 10 alkyl carbon atoms into a mixer to form a reaction mixture, a step S2 of sequentially injecting the reaction mixture into N number of batch reactors to perform a reaction such that the reaction is sequentially completed in the N number of batch reactors to semi-continuously manufacture reaction products, and a step S3 of semi-continuously moving the reaction products into a separation unit to remove unreacted alcohol, wherein N is an integer of 3 or greater.

Further embodiments are disclosed in the dependent claims.

### ADVANTAGEOUS EFFECTS

A manufacturing method of the present invention allows a plurality of batch reactors connected in parallel to be sequentially driven such that an entire reaction process is operated semi-continuously, so that it is possible to secure both the stability of a batch reactor and the efficiency of a semi-continuous process.

### BRIEF DESCRIPTION OF THE DRAWINGS

FIG. 1 is a process flow diagram illustrating a system for manufacturing an ester-based composition including a mixer, a supply control unit, a reaction unit, and a separation unit;
FIG. 2 is a process flow diagram illustrating a system for manufacturing an ester-based composition including a mixer, a supply control unit, a reaction unit, and a separation unit, wherein the supply control unit is provided inside the mixer;
FIGS. 3-5 are process flow diagrams each illustrating a system for manufacturing an ester-based composition indicating a path in which a catalyst may be injected,; and
FIG. 6 is a process flow diagram illustrating a system for manufacturing an ester-based composition including a mixer, a supply control unit, a reaction unit, a separation unit, and a trans-reaction unit.

### MODE FOR CARRYING OUT THE INVENTION

Hereinafter, the present invention will be described in more detail.

In a manufacturing method of the present invention, a polycarboxylic acid refers to a compound having two or more carboxylic acid groups, for example, a dicarboxylic acid, a tricarboxylic acid, or a tetracarboxylic acid. A polycarboxylic acid used in the present invention may have 2 to 5 carboxylic acid groups, 2 to 4 carboxylic acid groups, or 2 to 3 carboxylic acid groups. When a polycarboxylic acid has too many carboxylic acid groups, it may not easy to apply the polycarboxylic acid to the manufacturing method of the present invention due to a high molecular weight of the polycarboxylic acid itself. The polycarboxylic acid is preferably a dicarboxylic acid, a tricarboxylic acid, or a tetracarboxylic acid. The dicarboxylic acid may be one or more selected from the group consisting of a dicarboxylic acid which is of a linear type having 2 to 10 carbon atoms, a terephthalic acid, a phthalic acid, an isophthalic acid, and a cyclohexane dicarboxylic acid, and the tricarboxylic acid may be one or more selected from the group consisting of a citric acid, a trimellitate acid, and a cyclohexane tricarboxylic acid. The tetracarboxylic acid may be one or more selected from the group consisting of a benzenetetracarboxylic acid, a furantetracarboxylic acid, a cyclohexane tetracarboxylic acid, and a tetrahydrofuran tetracarboxylic acid. In addition, the polycarboxylic acid may not only include itself, but also include an anhydride or a derivative thereof.

In the manufacturing method of the present invention, it is preferably that alcohol having 3 to 10 alkyl carbon atoms is one or more selected from the group consisting of propanol, butanol, pentanol, hexanol, heptanol, octanol, nonanol and decanol, all of which are of a linear type or a branched type. In addition, the alcohol may be alcohol of a single type, or may be in the form of a mixture containing isomers having the same number of carbon atoms. For example, when the alcohol is alcohol having 3 alkyl carbon atoms, the alcohol may be 1-propanol or 2-propanol, or may be in the form of a mixture containing 1-propanol and 2-propanol in a predetermined ratio. When the alcohol is in the form of a mixture containing isomers having the same number of carbon number, the relative amount of each isomer is not particularly limited.

### System for manufacturing ester-based composition

The invention may be carried out in a system for manufacturing an ester-based composition, the system including a mixer in which a reaction mixture of a polycarboxylic acid and alcohol having 3 to 10 alkyl carbon atoms is formed, a reaction unit provided with N number of batch reactors connected in parallel in which an esterification reaction of the reaction mixture is performed, an inlet line for receiving the reaction mixture from the mixer, and an outlet line for discharging reaction products from the N number of batch reactors, a supply control unit for controlling the injection amount and the injection path of the reaction mixture such that the reaction mixture is sequentially supplied to the N number of batch reactors from the mixer to allow the reaction to be sequentially completed, and a separation unit for receiving the reaction products through the outlet line of the reaction unit and removing unreacted alcohol therefrom.

The manufacturing system is a system to be used for executing a manufacturing method of the present invention and includes a mixer 1, a supply control unit 2, a reaction unit 3, and a separation unit 4.

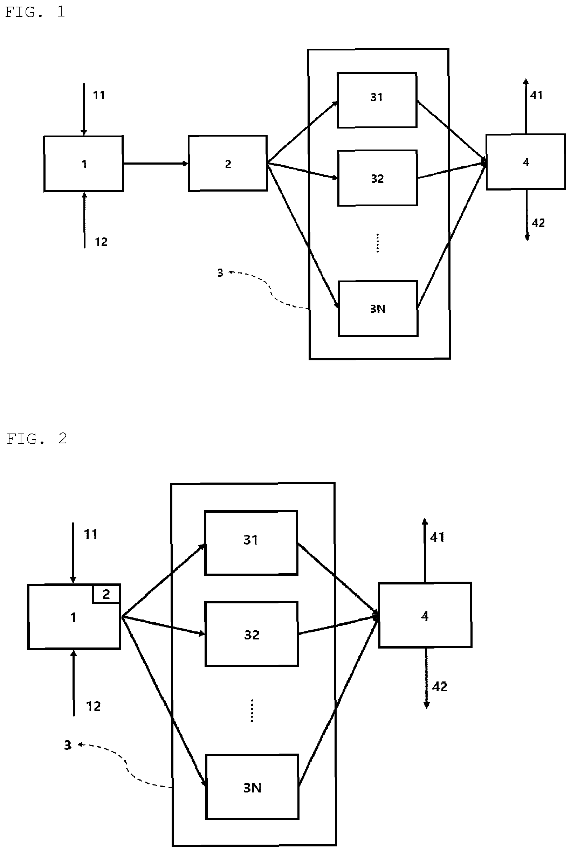

As shown in FIG. 1, the mixer 1 performs the mixing of a polycarboxylic acid 11 and alcohol 12 injected into the mixer, and a reaction mixture generated from the mixer passes through the supply control unit 2 to be sequentially injected into each of batch reactors 31 to 3N included in the reaction unit 3. When a reaction is completed in each reactor, reaction products are moved to the separation unit 4, and in the separation unit, unreacted alcohol 42 is removed and an ester-based composition 41 is finally obtained.

Particularly, the supply control unit 2 included in the manufacturing system serves to determine when to start an injection, how much to inject, and when to complete the injection for each reactor when the reaction mixture is sequentially injected into each reactor from the mixer, thereby enabling the sequential injection of the reaction mixture into each reactor connected in parallel and the discharge of the reaction products.

The supply control unit may be a separate unit connected to the mixer as shown in FIG. 1, and may be a unit included in the mixer as shown in FIG. 2. When the supply control unit is included in the mixer, the supply control unit may control the injection path and the injection amount of the reaction mixture directly discharged from the mixer.

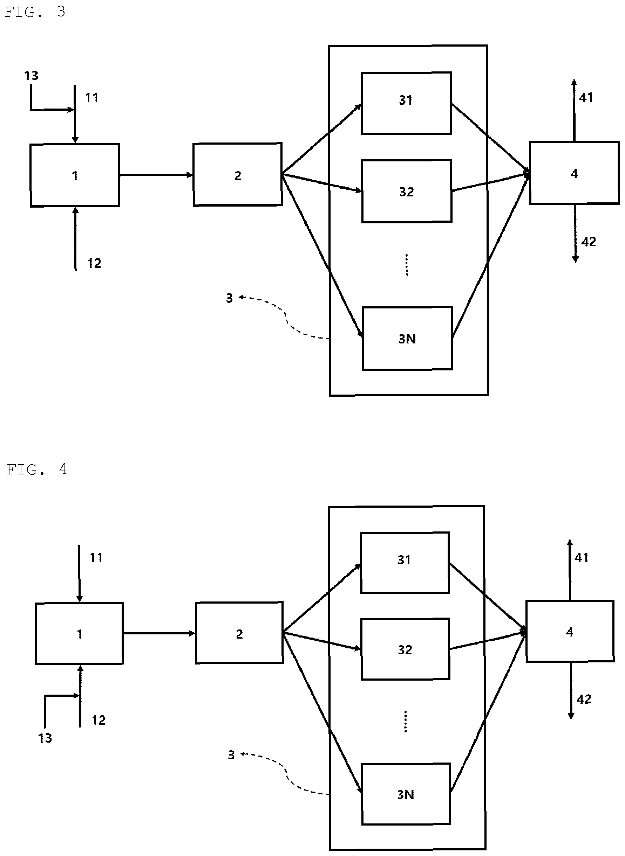

Also, as shown in FIG. 3, FIG. 4, or FIG. 5, in the manufacturing system, a catalyst 13 may be injected into the polycarboxylic acid, the alcohol, or the reaction mixture thereof.

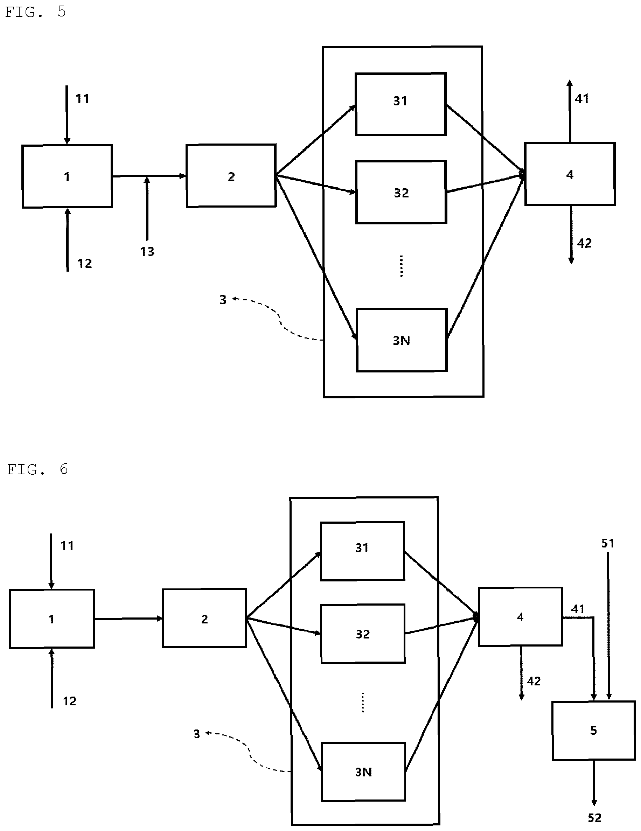

As shown in FIG. 6, the manufacturing system may further include a trans-reaction unit 5 for performing a trans-esterification reaction by adding alcohol having 3 to 10 alkyl carbon atoms to the reaction products from which the unreacted alcohol has been removed. Alcohol 52 injected from the trans-reaction unit is different from alcohol injected from the mixer, and may pass through the trans-reaction unit to manufacture an ester-based composition 51 including different ester-based compounds.

In addition, in the manufacturing system at least one among the N number of reactors may be provided with a gas-liquid separation column connected to an upper portion of the reactor and separating alcohol and water discharged through the upper portion of the reactor, a condenser for cooling a gas discharged through an upper line of the gas-liquid separation column, and a decanter for separating a liquid discharged through a lower line of the gas-liquid separation column and a liquid condensed in the condenser into different layers and recirculating the alcohol into the reactor.

As described above, when the reactor is provided with the gas-liquid separation column, the condenser, and the decanter, it is possible to increase the efficiency and economic feasibility of the reaction by re-liquefying alcohol vaporized during the reaction and re-injecting the re-liquefied alcohol into the reactor, and at the same time, it is possible to allow the reaction to proceed towards a forward reaction by removing water, which is a by-product of an esterification reaction, that is, allowing a high conversion rate to be achieved.

Also, the supply control unit in the manufacturing system controls one or more selected from the injection path and the injection flow rate of the reaction mixture to be changed according a predetermined time interval.

The supply control unit in the manufacturing system should determine the injection path and the injection flow rate of the reaction mixture such that N number of reactors may operate sequentially and set a time interval which is determined in consideration of the reaction duration, the total number of reactors, and the desired production amount, and the like. If the injection path and the injection flow rate of the reaction mixture is controlled at the predetermined time interval, at the time when the reaction products are all or almost discharged after the reaction is completed in each reactor, the reaction mixture is started to be injected back into a corresponding reactor, so that all of the reactors may be operated without being stopped, and the efficiency of the process may be increased.

In addition, the time interval set in the supply control unit may be 50%, 60%, 70%, 80%, 90% or greater, or 150%, 140%, 130%, 120%, 110% or less of a value obtained by dividing the reaction duration in one reactor by N. When the time interval is set in the above-described range, it is possible to minimize the loss of reactors not operating.

Meanwhile, the above-described reaction duration is a sum of the amount of time consumed for the reaction and the amount of time consumed for the injection of the reaction mixture and the discharge of the reaction products. For example, when 30 minutes are consumed for the reaction, and 15 minutes are consumed for each of the injection of the reaction mixture and the discharge of the reaction products, the reaction duration is 60 minutes. In this case, if there are four reactors, the injection time interval for each reactor is 15 minutes, so that the reaction mixture is injected into each reactor every 15 minutes.

### Method for manufacturing ester-based composition

The present invention provides a method for manufacturing an ester-based composition, the method including a step S1 of injecting a polycarboxylic acid and alcohol having 3 to 10 alkyl carbon atoms into a mixer to form a reaction mixture, a step S2 of sequentially injecting the reaction mixture into N number of batch reactors to perform a reaction such that the reaction is sequentially completed in the N number of batch reactors to semi-continuously manufacture reaction products, and a step S3 of semi-continuously moving the reaction products into a separation unit to remove unreacted alcohol, wherein N is an integer of 3 or greater.

### Mixing step (S1)

The manufacturing method of the present invention includes the step S1 of injecting a polycarboxylic acid and alcohol having 3 to 10 alkyl carbon atoms into a mixer to form a reaction mixture.

Specifically, the step S1 for forming the reaction mixture is a step of uniformly mixing the polycarboxylic acid and the alcohol having 3 to 10 carbon atoms in the mixer. In the present step, before the polycarboxylic acid and the alcohol having 3 to 10 carbon atoms, which correspond to reaction raw materials, are injected into a reactor, the polycarboxylic acid and the alcohol having 3 to 10 carbon atoms are uniformly pre-mixed in the mixer, so that it is possible to solve a non-uniform reaction which may occur when the raw materials are directly injected into the reactor. Particularly, since the reactor used in the present invention is a batch reactor, when reaction raw materials are not pre-mixed before being injected into the reactor, depending on a position inside the reactor, the non-uniformity of the raw materials may be greatly increased, and when stirring is performed poorly inside the reactor, some raw materials may be accumulated in particular, and thus, it may be difficult to secure uniform reaction duration and a uniform conversion rate. However, when reaction raw materials are pre-mixed and then injected, it is possible to obtain a substantially uniform reaction degree over the entire region of the reactor, and the reaction rate of each reactor may be maintained to be substantially uniform to secure the stability of the entire process.

In the manufacturing method of the present invention, the step S1 may further include a step in which the reaction mixture is heated to 50-200°C, preferably 60-190°C, more preferably 70-180°C. Since the reaction mixture is heated in the step S2 after the step S1 and then subjected to a reaction, when the reaction mixture is pre-heated and then injected into a reactor, the reaction mixture may be reacted easily and fast in the reactor. However, if an elevated temperature in the step S1 is too low, the effect of preheating before injection is poor. If heated to an excessively high temperature and injected into a reactor, the polycarboxylic acid and the alcohol having 3 to 10 alkyl carbon atoms are vaporized and the like, so that a uniform reaction may not rather proceed.

### Reaction step (S2)

The method for manufacturing an ester-based composition of the present invention includes the step S2 of sequentially injecting the reaction mixture into N number of batch reactors to perform a reaction such that the reaction is sequentially completed in the N number of batch reactors to semi-continuously manufacture reaction products.

In the case of a reaction process in which a typical batch reactor is used, although a large amount of reaction products could be stably manufactured at one time, the reactor is not operated during a process in which reaction raw materials are injected or the reaction products are discharged, so that there is a disadvantage in terms of the efficiency of the entire process. Therefore, the present inventors have invented a method for manufacturing an ester-based composition, the method in which a plurality of batch reactors are sequentially used, so that reaction products are semi-continuously manufactured while the stability of the batch reactor is still maintained.

Specifically, in the step S2 of the manufacturing method of the present invention, the reaction mixture is sequentially injected into the N number of batch reactors, and each reactor into which the reaction mixture is injected is heated to complete a reaction. After the reaction is completed, each reactor also sequentially discharges reaction products.

For example, the step S2 may be performed in the following manner:
1) A reaction mixture uniformly mixed in a mixer is injected into a first reactor, and after a predetermined amount of the reaction mixture is injected into the first reactor, the injection is stopped.
2) After the injection is stopped, the first reactor is heated to perform a reaction, and the mixer injects the reaction mixture into a second reactor.
3) After a predetermined amount of the reaction mixture is injected into the second reactor, the injection is stopped. After this point, the second reactor is heated to perform a reaction, and the mixer injects the reaction mixture into a third reactor.
4) N number of reactors sequentially manufacture reaction products in the above manner, and after the reaction mixture is injected into an N-th reactor, the reaction mixture is injected back into the first reactor. Also, reaction products manufactured after the reaction is completed are sequentially discharged in the same manner.

In the step S2, a time interval between the injection into each reactor, that is, a time interval of the sequential injection is 90% to 110%, preferably 100% of a value obtained by dividing the total reaction duration by the number of reactors. When the reaction mixture is injected into each reactor at the above interval, at the time when the reaction products are all or almost discharged after the reaction is completed in each reactor, the reaction mixture is started to be injected back into a corresponding reactor, so that all of the reactors may be operated without being stopped and the efficiency of the process may be increased.

The above-described reaction duration is a sum of the amount of time consumed for the reaction and the amount of time consumed for the injection of the reaction mixture and the discharge of the reaction products. For example, when 30 minutes are consumed for the reaction, and 15 minutes are consumed for each of the injection of the reaction mixture and the discharge of the reaction products, the reaction duration is 60 minutes. In this case, if there are four reactors, the injection time interval for each reactor is 15 minutes, so that the reaction mixture is injected into each reactor every 15 minutes.

In the manufacturing method of the present invention, in the step S2, the injection of the reaction mixture into a reactor, heating, a reaction, and the discharge of reaction products are all performed at the same time, so that at least one of a plurality of reactors should receive the reaction mixture, at least another one of the plurality of reactors should perform the reaction, and at least another one of the plurality of reactors should discharge the reaction products. Therefore, N is preferably an integer of 3 or greater.

Particularly, N may be an integer of 3 to 10, an integer of 3 to 7, or an integer of 3 to 5. If there are too many reactors, a variety of additional apparatuses are needed, including a control unit for controlling a reaction mixture to be injected into each reactor in order and reaction products to be discharged from each reactor. Furthermore, reaction duration per one reactor may be shorter than the sum of the amount of injection time of the raw materials injected into a reactor and the amount of discharge time of a reaction products, so that there may be time during which the reactor does not operate before receiving raw materials, which may result in adverse effects on productivity. In addition, the space required for the placement of each reactor becomes also excessive, which may be inefficient in terms of the costs for the entire process.

In the step S2 of the manufacturing method of the present invention, an esterification reaction of the polycarboxylic acid and the alcohol having 3 to 10 alkyl carbon atoms is performed. An esterification reaction refers to a reaction which a hydroxy group of alcohol and a carboxylic acid group of a polycarboxylic acid are reacted, thereby forming an ester bond. The esterification reaction of the step S2 may be performed at 130-250°C, preferably 140-240°C, more preferably 150-230°C. When an elevated temperature in the step S2 is lower than the above range, energy required for the reaction is not sufficiently supplied, so that the reaction may not proceed to a sufficient degree. When higher than the above range, vaporization of reaction mixture components, or the like occurs during the reaction as in the step S1, and thus, reaction products may not be manufactured to a sufficient amount.

### Separation step (S3)

The manufacturing method of the present invention includes the step S3 of semi-continuously moving the reaction products into a separation unit to remove unreacted alcohol.

Specifically, in the step S3, reaction products manufactured in each of the N number of batch reactors are semi-continuously moved to a separation unit, and then, unreacted alcohol is removed in the separation unit. As described above, as the injection of the reaction mixture into the N number of batch reactors may be sequentially performed, the discharge of the reaction products manufactured in each reactor may be also sequentially, or semi-continuously, performed.

The separation unit used in the step S3 may include one or more separation columns. Depending on the number of stages of the separation column included in the separation unit in the manufacturing method of the present invention, the composition ratio of a composition to be finally manufactured may vary. Those skilled in the art may appropriately adjust the number of stages of the separation column included in the separation unit according to the composition ratio or properties of the composition to be manufactured. In addition, the separation unit may include a purification tank of a drum type in addition to the separation column. The separation unit may remove the amount of unreacted alcohol included in the reaction products to a level of 30% or less, preferably 20% or less, more preferably 10% or less of the total. Since the unreacted alcohol is removed as described above, the physical properties of an ester-based composition to be manufactured may be uniform and excellent.

Typically, it is desirable that the separation column is operated continuously in terms of production management, and to this end, the reaction products discharged from each reactor may temporarily stay in a piece of equipment such as a tank before being injected into the separation column. The reaction products including the unreacted alcohol may stay in the equipment for about 0.1-10 hours, and the size of the equipment is not limited as long as the reaction products are stably and continuously supplied to the separation column.

### Catalyst addition step (S1-1 or S1-2)

The manufacturing method of the present invention may further include a step S1-1 of adding a catalyst to the reaction mixture between the step S1 and the step S2, or a step S1-2 of adding a catalyst to a polycarboxylic acid and alcohol having 3 to 10 alkyl carbon atoms before the step S1.

In the esterification reaction of alcohol and a carboxylic acid, a catalyst may be used, and when a catalyst is used, there is an advantage in that the reaction may be completed faster. The catalyst may be injected to a mixture of a polycarboxylic acid and alcohol, or to each of a polycarboxylic acid and alcohol before a mixture thereof is prepared. Particularly, it is preferable that the catalyst is added directly to alcohol in terms of the efficiency of the entire process.

The catalyst used in the manufacturing method of the present invention may be one or more selected from an acid catalyst such as sulfuric acid, hydrochloric acid, phosphoric acid, nitric acid, paratoluenesulfonic acid, methanesulfonic acid, ethanesulfonic acid, propanesulfonic acid, butanesulfonic acid, and alkyl sulfuric acid, a metal salt such as aluminum lactate, lithium fluoride, potassium chloride, cesium chloride, calcium chloride, iron chloride, and phosphoric acid, a metal oxide such as heteropoly acid, a natural/synthetic zeolite, a cation and anion exchange resin, and an organic metal such as tetraalkyl titanate and a polymer thereof, and may preferably be tetraalkyl titanate. As the tetraalkyl titanate, TiPT, TnBT, TEHT, or the like may be used, and it is preferable to use tetraalkyl titanate having an alkyl group same as the alkyl group of alcohol having 3 to 10 alkyl carbon atoms as a ligand. When a catalyst having the same alkyl group as a ligand is used, it is preferable because catalyst by-products which may be generated in a subsequent process are controlled or not generated.

The amount of a catalyst to be used may vary depending on the kind of the catalyst. In one example, a homogeneous catalyst may be used in an amount range of 0.001-5 wt%, 0.001-4 wt%, 0.01-3 wt%, or 0.01-2 wt% based on 100 wt% of the reaction mixture, and a heterogeneous catalyst may be used in an amount range of 5-200 wt%, 5-150 wt%, 10-150 wt%, or 20-150 wt% based on the total weight of the reaction mixture.

### Trans-reaction step (S4)

The manufacturing method of the present invention may further include a step S4 of injecting alcohol having 3 to 10 alkyl carbon atoms into the reaction products from which the unreacted alcohol is removed to perform a trans-esterification reaction, wherein the alcohol injected herein is different from the alcohol injected in the step S1.

Through the step S4, it is possible to manufacture a composition including two or more types of ester compounds. Those skilled in the art may select suitable alcohol according to the type of an ester compound to be included in the composition and perform a trans-esterification reaction. It is preferable that the step S4 is performed after the removal of unreacted alcohol. When the step S4 is performed before the removal of the unreacted alcohol, a trans-esterification reaction with newly injected alcohol may not be easily performed due to the remaining unreacted alcohol, and even the reaction is performed to a certain degree, the alcohol content is too high to deteriorate the efficiency of the reaction. Therefore, it is preferable that the amount of the unreacted alcohol included in the reaction products before the trans-esterification reaction is 10% or less.

### Description of the Reference Numerals or Symbols

1: Mixer
11: Injection path of polycarboxylic acid
12: Injection path of alcohol
13: Injection path of catalyst
2: Supply control unit
3: Reaction unit
31 to 3N: Each batch reactor (total of N reactors)
4: Separation unit
41: Movement path of ester-based composition from which unreacted alcohol is removed
42: Movement path of removed unreacted alcohol
5: Trans-reaction unit
51: Injection path of alcohol different from alcohol injected into 12
52: Movement path of ester-based composition which has been gone through trans-esterification reaction
92: Second recovery alcohol stream
93: Wastewater stream

## Claims

1. A method for manufacturing an ester-based composition, the method comprising:
a step S1 of injecting a polycarboxylic acid and alcohol having 3 to 10 alkyl carbon atoms into a mixer to form a reaction mixture;
a step S2 of sequentially injecting the reaction mixture into N number of batch reactors connected in parallel to perform a reaction such that the reaction is sequentially completed in the N number of batch reactors to semi-continuously manufacture reaction products, whereby the injection amount and the injection path of the reaction mixture is controlled by a supply control unit, which further controls one or more selected from the injection path and the injection flow rate of the reaction mixture to be changes according to a predetermined time interval. and
a step S3 of semi-continuously moving the reaction products into a separation unit to remove unreacted alcohol,
wherein N is an integer of 3 or greater.

2. The method of claim 1, further comprising
a step S1-1 of adding a catalyst to the reaction mixture between the step S1 and the step S2; or
a step S1-2 of adding a catalyst to a polycarboxylic acid and alcohol having 3 to 10 alkyl carbon atoms before the step S1.

3. The method of claim 2, wherein the catalyst is tetraalkyl titanate.

4. The method of claim 1, wherein the step S1 further comprises a step in which the reaction mixture is heated to 50-200°C.

5. The method of claim 1, wherein the temperature at which the reaction of the step S2 is performed is 130-250°C.

6. The method of claim 1, further comprising a step S4 of injecting alcohol having 3 to 10 alkyl carbon atoms into the reaction products from which the unreacted alcohol is removed to perform a trans-esterification reaction, wherein the alcohol injected in the step S4 is different from the alcohol injected in the step S1.

7. The method of claim 1, wherein at least one reactor among the N number of reactors comprises a gas-liquid separation column connected to an upper portion of the reactor and separating alcohol and water discharged through the upper portion of the reactor, a condenser for cooling a gas discharged through an upper line of the gas-liquid separation column, and a decanter for separating a liquid discharged through a lower line of the gas-liquid separation column and a liquid condensed in the condenser into different layers and recirculating the alcohol into the reactor. 9.

8. The method of claim 1, wherein the time interval is 50% to 150% of a value obtained by dividing the reaction duration in one reactor by N.

9. The method of claim 1, wherein the polycarboxylic acid is one or more selected from the group consisting of a dicarboxylic acid, a tricarboxylic acid, and a tetracarboxylic acid.

10. The method of claim 9, wherein
the dicarboxylic acid is one or more selected from the group consisting of a dicarboxylic acid which is of a linear type having 2 to 10 carbon atoms, a terephthalic acid, a phthalic acid, an isophthalic acid, a cyclohexane dicarboxylic acid, an anhydride thereof, and a derivative thereof,
the tricarboxylic acid is one or more selected from the group consisting of a citric acid, a trimellitate acid, a cyclohexane tricarboxylic acid, an anhydride thereof, and a derivative thereof, and
the tetracarboxylic acid is one or more selected from the group consisting of a benzenetetracarboxylic acid, a furantetracarboxylic acid, a cyclohexane tetracarboxylic acid, a tetrahydrofuran tetracarboxylic acid, an anhydride thereof, and a derivative thereof.

## Patentansprüche

1. Verfahren zum Herstellen einer Zusammensetzung auf Esterbasis, wobei das Verfahren umfasst:
einen Schritt S1 eines Injizierens einer Polycarbonsäure und eines Alkohols mit 3 bis 10 Alkylkohlenstoffatomen in einen Mischer, um eine Reaktionsmischung zu bilden;
einen Schritt S2 eines sequentiellen Injizierens der Reaktionsmischung in eine Anzahl N von Chargenreaktoren, die parallel miteinander verbunden sind, um eine Reaktion so durchzuführen, dass die Reaktion sequentiell in der Anzahl N von Chargenreaktoren zu halb-kontinuierlich hergestellten Reaktionsprodukten vervollständigt wird, wobei die Injektionsmenge und der Injektionsweg der Reaktionsmischung durch eine Versorgungssteuerungseinheit gesteuert wird, die ferner eines oder mehrere ausgewählt aus dem Injektionsweg und der Injektionsflussrate der Reaktionsmischung steuert, um gemäß einem vorgegebenen Zeitintervall geändert zu werden, und
einen Schritt S3 eines halb-kontinuierlichen Bewegens der Reaktionsprodukte in eine Trenneinheit, um nicht-umgesetzten Alkohol zu entfernen, wobei N eine ganze Zahl von 3 oder größer ist.

2. Verfahren nach Anspruch 1, weiter umfassend
einen Schritt S1-1 eines Zufügens eines Katalysators zu der Reaktionsmischung zwischen dem Schritt S1 und S2; oder
einen Schritt S1-2 eines Zufügens eines Katalysators zu einer Polycarbonsäure und einem Alkohol mit 3-10 Alkylkohlenstoffatomen vor dem Schritt S1.

3. Verfahren nach Anspruch 2, wobei der Katalysator Tetraalkyltitanat ist.

4. Verfahren nach Anspruch 1, wobei der Schritt S1 ferner einen Schritt umfasst, bei dem die Reaktionsmischung auf 50-200°C erwärmt wird.

5. Verfahren nach Anspruch 1, wobei die Temperatur, bei der die Reaktion von Schritt S2 durchgeführt wird, 130-250°C ist.

6. Verfahren nach Anspruch 1, weiter umfassend einen Schritt S4 eines Injizierens von Alkohol mit 3 bis 10 Alkylkohlenstoffatomen in die Reaktionsprodukte, von denen der nicht-umgesetzte Alkohol entfernt ist, um eine Umesterungsreaktion durchzuführen, wobei der in dem Schritt S4 injizierte Alkohol von dem in dem in Schritt S1 injizierten Alkohol verschieden ist.

7. Verfahren nach Anspruch 1, wobei wenigstens ein Reaktor unter der Anzahl von N Reaktoren eine Gas-Flüssig-Trennsäule, die mit einem oberen Bereich des Reaktors verbunden ist und Alkohol und Wasser, die durch den oberen Bereich des Reaktors abgegeben werden, getrennt werden, einen Kondensator zum Kühlen eines Gases, das durch eine obere Leitung der Gas-Flüssig-Trennsäule abgegeben wird, und einen Dekanter zum Abtrennen einer Flüssigkeit, die durch eine untere Leitung der Gas-Flüssig-Trennsäule abgegeben wird und einer Flüssigkeit, die in dem Kondensator kondensiert wird, in unterschiedliche Schichten und Rezirkulieren des Alkohols in den Reaktor umfasst.

8. Verfahren nach Anspruch 1, wobei das Zeitintervall 50% bis 150% eines Wertes ist, der Erhalten wird durch Teilen der Reaktionsdauer in einem Reaktor durch N.

9. Verfahren nach Anspruch 1, wobei die Polycarbonsäure eine oder mehrere ist, ausgewählt aus der Gruppe bestehend aus einer Dicarbonsäure, einer Tricarbonsäure und einer Tetracarbonsäure.

10. Verfahren nach Anspruch 9, wobei die Dicarbonsäure eine oder mehrere ist, ausgewählt aus der Gruppe bestehend aus einer Dicarbonsäure, die von einem linearen Typ mit 2 bis 10 Kohlenstoffatomen ist, einer Terephthalsäure, einer Phthalsäure, einer Isophthalsäure, einer Cyclohexandicarbonsäure, einem Anhydrid derselben und einem Derivat derselben,
die Tricarbonsäure eine oder mehrere ist, ausgewählt aus der Gruppe bestehend aus einer Citronensäure, einer Trimellitatsäure, einer Cyclohexantricarbonsäure, einem Anhydrid derselben und einem Derivat derselben, und
die Tetracarbonsäure eine oder mehrere ist, ausgewählt aus der Gruppe bestehend aus einer Benzoltetracarbonsäure, eine Furantetracarbonsäure, einer Cyclohexantetracarbonsäure, einer Tetrahydrofurantetracarbonsäure, einem Anhydrid derselben und einem Derivat derselben.

## Revendications

1. Procédé de fabrication d'une composition à base d'ester, le procédé comprenant :
une étape S1 consistant à injecter un acide polycarboxylique et un alcool ayant 3 à 10 atomes de carbone dans un mélangeur pour former un mélange de réaction ;
une étape S2 consistant à injecter séquentiellement le mélange de réaction dans un nombre N de réacteurs discontinus reliés en parallèle pour effectuer une réaction de telle sorte que la réaction est effectuée séquentiellement dans le nombre N de réacteurs discontinus pour fabriquer en semi-continu des produits de réaction, grâce à quoi la quantité d'injection et le trajet d'injection du mélange de réaction sont commandés par une unité de commande d'approvisionnement, laquelle commande en outre un ou plusieurs éléments sélectionnés parmi le trajet d'injection et le débit d'injection du mélange de réaction à changer selon un intervalle de temps prédéterminé, et
une étape S3 consistant à déplacer en semi-continu les produits de réaction dans une unité de séparation pour retirer l'alcool non réagi,
dans lequel N est un nombre entier de 3 ou plus.

2. Procédé selon la revendication 1, comprenant en outre
une étape S1-1 consistant à ajouter un catalyseur au mélange de réaction entre l'étape S1 et l'étape S2 ; ou
une étape S1-2 consistant à ajouter un catalyseur à un acide polycarboxylique et un alcool ayant 3 à 10 atomes de carbone avant l'étape S1.

3. Procédé selon la revendication 2, dans lequel le catalyseur est le titanate tétraalkyle.

4. Procédé selon la revendication 1, dans lequel l'étape S1 comprend en outre une étape dans laquelle le mélange de réaction est chauffé entre 50 et 200°C.

5. Procédé selon la revendication 1, dans lequel la température à laquelle la réaction de l'étape S2 est effectuée est comprise entre 130 et 250°C.

6. Procédé selon la revendication 1, comprenant en outre une étape S4 consistant à injecter un alcool ayant 3 à 10 atomes de carbone dans les produits de réaction d'où l'alcool non réagi est retiré pour effectuer une réaction de transestérification, dans lequel l'alcool injecté à l'étape S4 est différent de l'alcool injecté à l'étape S1.

7. Procédé selon la revendication 1, dans lequel un ou plusieurs réacteurs parmi le nombre N de réacteurs comprennent une colonne de séparation gaz-liquide reliée à une partie supérieure du réacteur pour séparer l'alcool et l'eau déchargée par la partie supérieure du réacteur, un condenseur pour refroidir un gaz déchargé par une conduite supérieure de la colonne de séparation gaz-liquide, et un décanteur pour séparer un liquide déchargé par une conduite inférieure de la colonne de séparation gaz-liquide et un liquide condensé dans le condenseur dans différentes couches et pour recirculer l'alcool dans le réacteur.

8. Procédé selon la revendication 1, dans lequel l'intervalle de temps est compris entre 50 % et 150 % d'une valeur obtenue en divisant la durée de réaction dans un réacteur par N.

9. Procédé selon la revendication 1, dans lequel l'acide polycarboxylique est un ou plusieurs éléments sélectionnés dans le groupe constitué d'un acide dicarboxylique, un acide tricarboxylique et un acide tétracarboxylique.

10. Procédé selon la revendication 9, dans lequel
l'acide dicarboxylique est un ou plusieurs éléments sélectionnés dans le groupe constitué d'un acide dicarboxylique qui est de type linéaire ayant 2 à 10 atomes de carbone, un acide téréphtalique, un acide phtalique, un acide isophtalique, un acide dicarboxylique de cyclohexane, un anhydride de ces derniers et un dérivé de ces derniers,
l'acide tricarboxylique est un ou plusieurs éléments sélectionnés dans le groupe constitué d'un acide citrique, un acide trimellitate, un acide tricarboxylique de cyclohexane, un anhydride de ces derniers et un dérivé de ces derniers , et
l'acide tétracarboxylique est un ou plusieurs éléments sélectionnés dans le groupe constitué d'un acide tétracarboxylique de benzène, un acide tétracarboxylique de furane, un acide tétracarboxylique de cyclohexane, un acide tétracarboxylique de tétrahydrofurane, un anhydride de ces derniers et un dérivé de ces derniers.
